## Europäisches Patentamt

## European Patent Office

## Office européen des brevets

(11) Veröffentlichungsnummer: **0 080 518**
**B1**

(12) # EUROPÄISCHE PATENTSCHRIFT

(45) Veröffentlichungstag der Patentschrift:
15.10.86

(51) Int. Cl.⁴: **A 61 K 35/26**

(21) Anmeldenummer: **81109909.2**

(22) Anmeldetag: **26.11.81**

(54) Verfahren zur Extraktion der Thymusdrüse.

(43) Veröffentlichungstag der Anmeldung:
08.06.83 Patentblatt 83/23

(45) Bekanntmachung des Hinweises auf die Patenterteilung:
15.10.86 Patentblatt 86/42

(84) Benannte Vertragsstaaten:
AT BE CH DE FR GB IT LI LU NL SE

(56) Entgegenhaltungen:
DE-A-2 110 436
DE-A-2 819 131
DE-B-1 214 831
FR-A-1 294 225
GB-A-339 943

CHEMICAL ABSTRACTS, Band 55, Nr. 6, 20. März 1961, Spalte 5772e, Columbus, Ohio, USA I. POTOP et al.: "The effect of certain fractions isolated from thymus upon the development of experimental tumors"
CHEMICAL ABSTRACTS, Band 74, 1971, Seite 249, Nr. 74202s, Columbus, Ohio, USA E. JUVINA: "Action of thymic lipid and protein extract on liver phosphorus and alkaline phosphatease in the Walker-tumorbearing rat"

(73) Patentinhaber: **Ollendiek, Heinrich, Dr. med.,
Frankfurter Strasse 45, D-6350 Bad Nauheim (DE)**

(72) Erfinder: **Ollendiek, Heinrich, Dr. med.,
Frankfurter Strasse 45, D-6350 Bad Nauheim (DE)**

(74) Vertreter: **Zinngrebe, Horst, Dr.rer.nat.,
Saalbaustrasse 11, D-6100 Darmstadt (DE)**

**Beschreibung**

Die Erfindung betrifft ein Verfahren zur Gewinnung eines therapeutisch wirksamen Wirkstoffes aus der Thymusdrüse. Aus der Thymusdrüse kann man folgende Substanzen extrahieren:

1) Die Thymushormone
2) Gewebsenzyme
3) Unspezifische Eiweißkörper.

Von den Thymushormonen weiß man besonders durch die Untersuchungen Goldsteins, daß sie das lymphatische System auch des menschlichen Körpers stimulieren. Dadurch wird unter anderem auch die Abwehrmöglichkeit des menschlichen Körpers gegenüber dem Krebs angeregt.

Von den Gewebsenzymen der Thymusdrüse weiß man seit jüngster Zeit, daß sie direkt tumorschädlich sind und die Tumorzelle so verändern kann, daß diese der körpereigenen Abwehr besser zugänglich wird.

Von den unspezifischen Eiweißkörpern ist bekannt, daß sie nach Injektion beim Menschen unspezifische Abwehrreaktionen stimulieren. Außerdem wird auch die körpereigene Produktion von Interferon dadurch angeregt.

Wenn man aufgrund dieser Kenntnisse einen Thymusextrakt herstellen und therapeutisch verwenden will, sind folgende Punkte zu berücksichtigen:

Das Präparat muß verträglich sein, es muß in wirksamer Dosierung applizierbare sein, es muß ferner in ausreichender Menge herstellbar sein, das Herstellungsverfahren darf für die allgemeine Anwendung nicht zu teuer sein, und es sollten möglichst alle wirksamen Komponenten verwendet werden können. In bezug auf die Extraktion der Thymusdrüsen-Wirkstoffe ergeben sich nun folgende Probleme: '

Im Hinblick auf die Verträglichkeit und damit ausreichender Dosierbarkeit verursachen die unspezifischen Eiweißkörper des Thymus-Gesamtektraktes die meisten Schwierigkeiten. Sie lösen bei wirksamer Dosierung meistens heftige lokale Reaktionen aus und können auch zu anaphylaktischen Reaktionen führen. Nun führt aber eine gründliche Eliminierung dieser Eiweiß-Körper dazu, daß gleichzeitig ein Großteil der Hormone mit eliminiert wird und daß praktisch alle therapeutisch wertvollen Enzyme zerstört werden. Es gibt auf dem Markt Präparate, die nach diesem Prinzip hergestellt sind.

Da man daher auf die Enzyme weitgehend verzichten zu müssen glaubte, hat man versucht, Thymus-Hormone synthetisch herzustellen. Da jedoch die Enzyme der Thymusdrüse wesentlich komplizierter aufgebaut sind als die Hormone, bereitet eine Synthese erhebliche Schwierigkeiten.

Dagegen hat Sandberg in Schweden ein einfaches Herstellungsverfahren angegeben: die steril entnommenen Thymusdrüsen werden unter Zusatz von destilliertem Wasser grob homogenisiert und 48 Stunden kühl abgestellt. Dann wird der Überstand entnommen und das Sediment verworfen. In einem auf diese Weise gewonnenen Präparat sind Hormone, Enzyme und unspezifische Eiweißkörper enthalten. Diese Präparat hat sich bei der Behandlung von Autoimmunerkrankungen, Arthrosen und zum Teil auch bei der chronischen Polyarthritis als recht wirksam erwiesen. Bei der Behandlung von bösartigen Tumoren erwies es sich aber in den allermeisten Fällen als nicht wirksam genug. Es kam über die Rolle eines zusätzlichen Adjuvans nicht hinaus. Eine wesentliche Erhöhung der Dosierung ist wegen den Nebenwirkungen nicht möglich, sodaß eine Steigerung der Wirkung in dieser Richtung nicht möglich war.

Aus DE—B 1 214 831 ist ein Verfahren zur Herstellung von Thymusextrakten bekannt, die zur Induzierung immunologischer Fähigkeit in lebenden Organismen wertvoll sind, bei dem man Thymus von jungen Kälbern isoliert, den Thymus in einem Gewebskulturmedium, beispielsweise in gepufferter Hanks-Lösung, inkubiert und die überstehende Flüssigkeit sammelt.

Über den Einfluß von Thymusextrakten auf die Entwicklung von experimentellem Tumor bei Tieren ist in Chemical Abstracts, Band 55, Nr. 6, 20. März 1961, Spalte 5772e, sowie in Chemical Abstracts, Band 74, 1971, Seite 249, Nr. 74202s, berichtet worden.

Zur Unterdrückung des Wachstums von Tumorzellen ist aus DE—A 2 819 131 ein Mischpräparat aus foetalem und jugendlichem Thymus bekannt.

In DE—A 2 110 436 wird ein Verfahren zur Herstellung eines Thymusextraktes mit antileukopenischer Wirkung beschrieben, gemäß dem man zerkleinerte Thymusteilchen eines jungen Kalbes mit einer lysierend wirkenden Lösung behandet, nach mehrmaliger Behandlung bei verschiedenen Temperaturen die Flüssigkeit von der Mischung abschleudert und diesen Vorgang erneut bei verschiedenen Temperaturen wiederholt. Ferner werden spezielle Verfahren in der FR—A 1 294 225 und in der GB—A 339 943 erläutert.

Demgegenüber liegt der Erfindung folgendes Ziel zugrunde: Das Präparat muß bei wesentlich höherer Wirkstoffkonzentration verträglicher sein, die Ausbeute des Rohmaterials muß wesentlich besser sein, um die höheren Herstellungskosten weitgehend abzufangen, es muß die Hormone und Enzyme möglichst in originaler Konzentration enthalten, weil diese beiden Komponenten sich in ihrer Wirksamkeit ergänzen, und es muß der Anteil der unspezifischen Eiweißkörper schonend soweit reduziert werden, daß es zu keinem wesentlichen Verlust der erwünschten Wirksubstanzen kommt, wobei aber gleichzeitig die Verträglichket des Präparats soweit verbessert werden soll, daß es in ausreichender Dosierung verabreicht werden kann.

Dazu wird das Verfahren zur Gewinnung von therapeutisch wirksamen Wirkstoffen aus der Thymusdrüse von frisch geschlachteten Kälbern durch Wasserextraktion erfindungsgemäß so geführt, daß die Thymusdrüse von etwa 12—16 Wochen alten Kälbern unter sterilen Kauteln entnommen, die entnommene Masse auf tiefe Temperaturen von wenigstens −30°C abgekühlt wird, nach dem Auftauen unter Zusatz von destilliertem Wasser homogenisiert wird, die homogenisierte Flüssigkeit einige Tage bei etwa +4°C abgestellt und anschließend schnell zentrifugiert und gefiltert wird, daß der Überstand aus dem Sediment des Filtrats dekantiert, einige Tage bei ca. +4°C abgestellt und erneut schnell zentrifugiert und gefiltert wird, und der Überstand aus dem zweiten Filtrat zu einem applizierbaren Präparat verarbeitet wird.

Eine besonders optimale Verfahrensführung sieht vor:

In einem ersten Schritt wird die Thymusdrüse von frisch geschlachteten, gesunden, etwa 12 bis 16 Wochen alten Kälbern unter sterilen Kauteln entnommen. Um die Grundsubstanz und die Fasern hinreichend zu zerkleinern empfiehlt es sich, die entnommene Masse durch einen üblichen Fleischwolf zu zerkleinern.

Die zerkleinerte Masse wird dann in einem zweiten und wesentlichen Verfahrensschritt auf tiefe Temperaturen eingefroren, und zwar bis wenigstens −30°C. Die tiefgekühlte Masse kann bei dieser Temperatur je nach Bedarf eine kürzere oder längere Zeitspanne gelagert werden. Durch das Tiefkühlen werden die Zellwände zerstört und die in den Zellen eingeschlossenen Organellen sowie die Enzyme freigesetzt.

Danach wird die eingefrorene Masse aufgetaut und im Überschuß mit aqua dest. versetzt, wobei sich ein Volumenverhältnis von einem Volumenteil Masse zu drei Volumenteilen Wasser empfiehlt. Die aufgeschlemmte Masse wird durch einen Mixer intensiv homogenisiert. Die auf diese Weise gewonnene trübe Flüssigkeit wird, etwa in einem Kühlschrank, einige Tage bei ca. +4°C stehengelassen. Während dieser Zeit tritt eine Autolyse ein, bei der die freigesetzten Enzyme die langen Eiweißketten zum Teil abbauen. Die Zeitspanne, während der die Flüssigkeit stehengelassen wird, ist für die Wirksamkeit des fertigen Präparates ein kritischer Parameter; praktische Versuche haben gezeigt, daß ein Zeitraum von 4—5 Tagen zu optimalen Ergebnissen führt.

Die trübe Flüssigkeit wird sodann in einer schnellen Zentrifuge mit einer Leistung oberhalb 20.000 g abzentrifugiert, so daß die Zelltrümmer, die großen, verbliebenen Eiweißmoleküle und die meisten Bakterien und Pilze sich im Sediment sammeln. Nach etwa zehn Minuten Zentrifugierzeit wird der Überstand über dem Sediment durch ein Gazefilter gegeben, der Filterrückstand und das Sediment werden verworfen.

Der gewonnene Überstand wird erneut einige Tage bei ca. +4°C stehengelassen, um die Autolyse weiter fortschreiten zu lassen. Auch hier empfiehlt sich ein Zeitraum von 4—5 Tagen für das Abstellen.

Danach wird die Flüssigkeit erneut solange zentrifugiert, bis der Überstand stark opalescierend, jedenfalls nicht mehr trüb ist. Das Sediment wird wieder verworfen. Der Überstand wird dann zu einem applizierbaren Präparat in üblicher Weise weiterverarbeitet, also etwa in Einmal-Spritzen aufgezogen oder zur Lagerung eingefroren oder zur Gewinnung der Trockensubstanz gefriergetrocknet.

Das auf diese Weise gewonnene Präparat ist wesentlich konzentrierter als bislang bekannt, kann außerdem in größerer Menge appliziert werden, und zwar intramuskulär oder subcutan. Das Präparat ist überraschend wirksam bei seiner Anwendung als Klistier. Diese Anwendung hat den Vorteil, daß sie mit wesentlich weniger Nebenwirkungen behaftet ist als bei der Injektion und daher noch höher dosiert werden kann.

Die Vorteile und die Wirkungen des erfindungsgemäß gewonnenen Präparates bestehen nach eingehenden Untersuchungen bei der Behandlung von Krebserkrankungen im folgenden:

1) Bei Anwendung ohne Zusatz-Therapie in vielen Fällen Stabilisierung des Tumors oder sogar seine Rückbildung, und

2) in Kombination mit Bestrahlung oder Chemotherapie deutlich bessere Wirksamkeit der konventionellen Behandlung und Reduzierung der damit gekoppelten unerwünschten Nebenwirkungen.

**Patentansprüche**

1. Verfahren zur Gewinnung von therapeutisch wirksamen Wirkstoffen aus der Thymusdrüse von frisch geschlachteten Kälbern durch Wasserextraktion, dadurch gekennzeichnet, daß die Thymusdrüse von etwa 12—16 Wochen alten Kälbern unter sterilen Kauteln entnommen, die entnommene Masse auf tiefe Temperaturen von wenigstens −30°C abgekühlt wird, nach dem Auftauen unter Zusatz von destilliertem Wasser homogenisiert wird, die homogenisierte Flüssigkeit einige Tage bei etwa +4°C abgestellt und anschließend schnell abzentrifugiert und gefiltert wird, daß der Überstand über dem Sediment des Filtrats dekantiert, einige Tage bei ca. +4°C abgestellt und erneut schnell zentrifugiert und gefiltert wird, und der Überstand aus dem zweiten Filtrat zu einem applizierbaren Präparat verarbeitet wird.

2. Verfahren nach Anspruch 1, dadurch gekennzeichnet, daß die den Kälbern entnommene Thymusdrüse vor dem Einfrieren durch einen Fleischwolf zerkleinert wird.

3. Verfahren nach Anspruch 1 oder 2, dadurch gekennzeichnet, daß einem Volumenteil aufgetauter Drüsenmasse drei Volumenteile aqua dest. zugesetzt werden.

4. Verfahren nach einem der vorstehenden Ansprüche, dadurch gekennzeichnet, daß die homogenisierte Flüssigkeit etwa 4—5 Tage bei ca. +4°C abgestellt wird.

5. Verfahren nach einem der vorstehenden Ansprüche, dadurch gekennzeichnet, daß der Überstand aus dem ersten Filtrat etwa 4—5 Tage abgestellt wird.

6. Verfahren nach einem der vorstehenden Ansprüche, dadurch gekennzeichnet, daß mit einer Zentrifugierleistung oberhalb 20.000 g abzentrifugiert wird.

7. Verfahren nach einem der vorstehenden Ansprüche, dadurch gekennzeichnet, daß das zweite Zentrifugieren solange ausgeführt wird, bis der Überstand stark opalescierend ist.

**Revendications**

1. Procédé permettant d'extraire, par déshydratation, des principes à action thérapeutique du thymus de veaux fraîchement abattus, caractérisé en ce que le thymus de veaux de 12 à 16 semaines est prélevé en environnement stérile, la masse prélevée est congelée à au moins −30°C puis, après décongélation, homogénéisée en présence d'eau distillée, le liquide homogénéisé est conservé quelques jours à environ +4°C puis centrifugé à vitesse élevée et filtré, et en ce que le surnageant est séparé du dépôt du filtrat par décantation, conservé quelques jours à environ +4°C, à nouveau centrifugé à vitesse élevée et filtré, et le surnageant du second filtrat est transformé en une préparation administrable.

2. Procédé conforme à la revendication 1, caractérisé en ce que le thymus prélevé sur les veaux est fragmenté dans un hachoir à viande avant d'être congelé.

3. Procédé conforme à la revendication 1 ou 2, caractérisé en ce que trois parties en volume d'eau distillée sont ajoutées à une partie en volume de masse de thymus décongelée.

4. Procédé conforme à l'une quelconque des revendications précédentes, caractérisé en ce que le liquide homogénéisé est mis à reposer pendant environ 4 à 5 jours à environ +4°C.

5. Procédé conforme à l'une quelconque des revendications précédentes, caractérisé en ce que le surnageant provenant du premier filtrat est mis à reposer pendant environ 4 à 5 jours.

6. Procédé conforme à l'une quelconque des revendications précédentes, caractérisé en ce que la centrifugation s'effectue à une puissance supérieure à 20.000 g.

7. Procédé conforme à l'une quelconque des revendications précédentes, caractérisé en ce que la seconde centrifugation est poursuivie jusqu'à obtention d'un surnageant très opalescent.

**Claims**

1. A method of extraction of therapeutically effective hormones from the thymus glands of freshly slaughtered calves by extraction by water, characterized in that the thymus glands of calves of about 12—16 weeks old are removed under sterile precautions, the substance removed is cooled down to low temperatures of at least −30°C, after thawing is homogenized with the addition of distilled water, the homogenized liquid is set aside for a few days at about +4°C and subsequently centrifuged rapidly and filtered, that the supernatant above the sediment from the filtrate is decanted, is set aside for a few days at about +4°C and again centrifuged rapidly and filtered, and the supernatant from the second filtrate is processed into an applicable preparation.

2. A method as in Claim 1, characterised in that the thymus glands removed from the calves are comminuted before the freezing by a mincer.

3. A method as in Claim 1 or 2, characterized in that to one part by volume of thawed gland substance are added three parts by volume of aqua dest.

4. A method as in one of the preceding Claims, characterized in that the homogenized liquid is set aside for about 4—5 days at about +4°C.

5. A method as in one of the preceding Claims, characterized in that the supernatant from the first filtrate is set aside for about 4—5 days.

6. A method as in one of the preceding Claims, characterized in that the centrifuging is effected at a centrifuging power above 20.000 g.

7. A method as in one of the preceding Claims, characterized in that the second centrifuging is carried on until the supernatant is strongly opalescent.